# EUROPEAN PATENT APPLICATION

(11) **EP 2 518 654 A1**
(43) Date of publication of application: **31.10.2012**
(21) Application number: 12166001.3
(22) Date of filing: 27.04.2012
(51) Int. Cl.: G06F 19/00

(54) **Measuring device, communication device, monitoring system and program**

(30) Priority: 27.04.2011 JP 2011099015
(71) Applicant: Arkray, Inc., Minami-ku Kyoto-shi Kyoto 601-8045 (JP)
(72) Inventor: Kai, Akinori, Kyoto, Kyoto 6020008 (JP); Wada, Atsushi, Kyoto, Kyoto 6020008 (JP); Asega, Asuka, Kyoto, Kyoto 6020008 (JP)
(74) Representative: MacDougall, Alan John Shaw

(57) **Abstract**

A measuring device in a monitoring system including a monitoring device for monitoring data received via a network is provided. The measuring device includes a measurer for measuring the data, a data transmitter for sending the data to a communication device that performs communication with the monitoring device, a forbidding condition determiner for determining whether or not a predetermined forbidding condition is satisfied, and a transmission forbidder for forbidding transmission of the data to the communication device or to the monitoring device when the forbidding condition determiner determines that the predetermined forbidding condition is satisfied.

## Description

### BACKGROUND OF THE INVENTION

### 1. Field of the Invention:

The present invention relates to a monitoring system in which measurement result data are sent to a monitoring server for monitoring at the monitoring server. The invention also relates to a measuring device for performing measurement in such a monitoring system, a communication device for sending data in such a monitoring device, and a program for controlling the measuring device or the communication device.

### 2. Description of the Related Art:

Portable measuring devices which allow patients to measure various data by themselves have conventionally been developed. For instance, diabetics need to measure their blood glucose levels e.g. after a meal or before bedtime. Blood glucose level measuring devices are known as a portable measuring device which allows diabetics to easily perform self-measurement of the blood glucose level. Some of the blood glucose level measuring devices can send data including the measured blood glucose level (hereinafter referred to as "blood glucose-related data") to a monitoring server via a network.

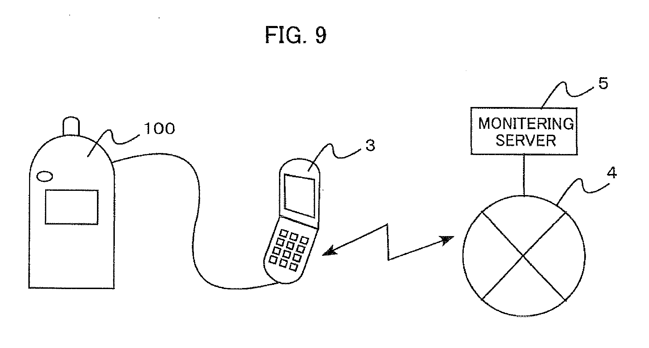

Fig. 9 is a view for describing a blood glucose level measuring device which can perform communication by using a communication device and can be connected to a monitoring server via a network.

The blood glucose level measuring device 100 shown in Fig. 9 is a device for measuring a blood glucose level. The blood glucose level measuring device 100 is connectable to a communication device 3 via a cable. The blood glucose-related data including the blood glucose level measured by the blood glucose level measuring device 100 is sent to the communication device 3 when the blood glucose level measuring device 100 is connected to the communication device 3 via a cable or when a predetermined operation is performed after the connection. The communication device 3 sends the blood glucose-related data to a monitoring server 5 via a network 4. The blood glucose-related data are stored in the monitoring server 5. The patient or the patient's doctor can check the stored blood glucose-related data by making access to the monitoring server 5.

When a mobile phone or the like is used as the communication device 3, the patient can send the blood glucose-related data to the monitoring server 5 from the site where the measurement is performed. In this case, therefore, the patient does not need to bring the blood glucose level measuring device 100 to a personal computer or the like and connect the blood glucose level measuring device 100 to the computer in order to make access to the network 4.

With some types of mobile phones, the international roaming service is available. By using the international roaming service, the patient can send the blood glucose-related data to the monitoring server 5 even when the patient is in a foreign country. However, since the communication charge using the international roaming service is generally high, sending blood glucose-related data when staying in a foreign country leads to high mobile phone charge. When the patient is under contract for a flat-rate billing plan, the patient may not worry about the charge for each communication. However, in most of such flat-rate billing plans, the user is to be charged an additional charge for communication using the international roaming service. If the patient uses the international roaming service without knowing this fact, the patient will be charged an unexpectedly high communication charge.

A similar problem can occur when the patient is in contract for a billing plan with which the communication charge varies depending on the time at which the communication is performed. For instance, if the patient sends blood glucose-related data by mistake in a time period to which discount is not applied, the patient will be charged unexpectedly high communication charge.

Further, a similar problem can occur when communication is performed using a communication device other than a mobile phone. For instance, such a problem can occur when the communication device is a smartphone or a personal computer. Moreover, a similar problem can occur when the measuring device is a device other than a blood glucose level measuring device 100, such as a blood analyzer, a urine analyzer, an electrocardiogram monitor or a blood pressure measuring device. In particular, such a problem can occur in the case where a blood pressure measuring device is kept attached to a patient' s body to constantly measure the blood pressure. In this case, the measuring device is kept connected to a communication device and the measurement result is sent automatically every time the measurement is performed. Thus, the user easily forgets the fact that data transmission is being performed and may forget to care about the charge for communication using the international roaming service or communication in a time period to which discount is not applied. In this case again, the patient will be charged unexpectedly high communication charge.

The present invention is conceived under the circumstances described above. It is therefore an object of the present invention to provide a monitoring system capable of preventing unexpectedly high communication charge resulting from data transmission to a monitoring server.
Patent Document 1: JP-A-2004-62285

### SUMMARY OF THE INVENTION

The present invention has been proposed under the circumstances described above. It is therefore an object of embodiments of the invention to provide a monitoring system that prevents the user from being charged unexpectedly high communication charge due to data transmission to a monitoring server.

To solve the above-described problems, the present invention takes the following technical measures.

According to a first aspect of the present invention, there is provided a measuring device in a monitoring system including a monitoring device for monitoring data received via a network. The measuring device comprises: a measurer for measuring the data; a data transmitter for sending the data to a communication device that performs communication with the monitoring device; a forbidding condition determiner for determining whether or not a predetermined forbidding condition is satisfied; and a transmission forbidder for forbidding transmission of the data to the communication device or to the monitoring device when the forbidding condition determiner determines that the predetermined forbidding condition is satisfied.

The "measuring device" in the present invention refers to devices that perform measurement and output the measurement results as data. Examples of such a measuring device include devices which detect a predetermined component in a sample and output the amount or proportion as measurements, such as a blood glucose level measuring device, a blood analyzer or a urine analyzer. An electrocardiogram monitor that measures the electrical activity of the heart and a blood pressure measuring apparatus are also included in the "measuring device". Further, the "measuring device" is not limited to medical devices, and a sugar content meter for measuring the sugar content in e.g. fruit juice or a position measuring device for obtaining positional information using GPS (Global Positioning System) are also included in the "measuring device".

The "communication device" in the present invention refers to devices that perform communication via a network. Examples of such a communication device include a mobile phone, a smartphone, a mobile information terminal, a laptop personal computer, a desktop personal computer and a fixed phone.

In a preferred embodiment of the present invention, the communication device includes a first storage for storing the data sent by the data transmitter, and the measuring device further comprises: a second storage for storing the data measured by the measurer; a selector for selecting to store the data in the first storage only, in the second storage only, or in both the first storage and the second storage; and a transmission instructor for instructing the communication device to send the data to the monitoring device. The transmission forbidder forbids the transmission instructor from instructing to send the data when storing the data in the first storage is set by the selector. The transmission forbidder forbids the data transmitter from sending the data when storing the data in the second storage only is set by the selector.

In a preferred embodiment of the present invention, the measuring device further comprises a sensor mount portion for mounting a sensor to which a measurement object is to be applied, and a sensor detector for detecting mounting and dismounting of the sensor to and from the sensor mount portion. The selector performs a selection operation in accordance with mounting or dismounting of the sensor detected by the sensor detector.

In a preferred embodiment of the present invention, the measuring device further comprises a service state checker for checking a communication service state of the communication device. The forbidding condition determiner determines that the predetermined forbidding condition is satisfied when the service state checker finds that a predetermined service is to be performed.

In a preferred embodiment of the present invention, the predetermined service is international roaming.

In a preferred embodiment of the present invention, the transmission instructor sends to the communication device an address of the monitoring device as well in instructing the communication device to send the data.

In a preferred embodiment of the present invention, the communication device includes a network detector for detecting communication networks available for performing communication with the monitoring device, and the measuring device further comprises: a network information receiver for receiving a plurality of pieces of network information each representing a respective one of the communication networks detected by the network detector; a display unit for displaying the pieces of network information; a network selector for selecting one from the pieces of network information displayed on the display unit; a network setter for setting the communication network represented by the piece of information selected by the network selector as a communication network for the communication device to perform communication with the monitoring device; a sensor mount portion for mounting a sensor to which a measurement object is to be applied; and a sensor detector for detecting mounting and dismounting of the sensor to and from the sensor mount portion. The network selector performs a network selection operation in accordance with mounting or dismounting of the sensor detected by the sensor detector.

In a preferred embodiment of the present invention, the measuring device further comprises a forbiddance-cancelling condition determiner for determining whether or not a predetermined forbiddance-cancelling condition is satisfied. The transmission forbidder forbids transmission of the data to the communication device or to the monitoring device when the forbidding condition determiner determines that the predetermined forbidding condition is satisfied and the forbiddance-cancelling condition determiner determines that the predetermined forbiddance-cancelling condition is not satisfied.

According to a second aspect of the present invention, there is provided a communication device for performing communication with a monitoring device for monitoring data received via a network in a monitoring system. The communication device comprises: a data obtainer for obtaining measurement results measured by a measuring device as the data; a transferer for sending the data to the monitoring device when the data obtainer obtains the data; a forbidding condition determiner for determining whether or not a predetermined forbidding condition is satisfied; and a transmission forbidder for forbidding the transferer from sending the data to the monitoring device when the forbidding condition determiner determines that the predetermined forbidding condition is satisfied.

In a preferred embodiment of the present invention, the communication device further comprises a service state checker for checking a communication service state. The forbidding condition determiner determines that the predetermined forbidding condition is satisfiedwhen the service state checker finds that a predetermined service is to be performed.

In a preferred embodiment of the present invention, the predetermined service is international roaming.

In a preferred embodiment of the present invention, the communication device further comprises a forbiddance-cancelling condition determiner for determining whetheror nota predeterminedforbiddance-cancelling condition is satisfied. The transmission forbidder forbids the transferer from sending the data to the monitoring device when the forbidding condition determiner determines that the predetermined forbidding condition is satisfied and the forbiddance-cancelling condition determiner determines that the predetermined forbiddance-cancelling condition is not satisfied.

In a preferred embodiment of the present invention, the monitoring device is provided for each of a plurality of kinds of data. The communication device further comprises a data discriminator for discriminating among the plurality of kinds of data. The transferer sends the data to the monitoring device corresponding to the kind of the data discriminated by the data discriminator.

According to a third aspect of the present invention, there is provided a monitoring system comprising the monitoring device and the measuring device provided according to the first aspect of the present invention or the communication device provided according to the second aspect of the present invention.

According to a fourth aspect of the present invention, there is provided a program for controlling a computer of a measuring device for measuring data, in a monitoring system including a monitoring device for monitoring the data received via a network. The program is designed to cause the computer to function as: a data transmitter for sending the data to a communication device that performs communication with the monitoring device; a forbidding condition determiner for determining whether or not a predetermined forbidding condition is satisfied; and a transmission forbidder for forbidding transmission of the data to the communication device or to the monitoring device when the forbidding condition determiner determines that the predetermined forbidding condition is satisfied.

According to a fifth aspect of the present invention, there is provided a program for controlling a computer of a communication device for performing communication with a monitoring device for monitoring data received via a network in a monitoring system. The program is designed to cause the computer to function as: a transferer for sending the data to the monitoring device upon receiving the data; a forbidding condition determiner for determining whether or not a predetermined forbidding condition is satisfied; and a transmission forbidder for forbidding the transferer from sending the data to the monitoring device when the forbidding condition determiner determines that the predetermined forbidding condition is satisfied.

According to the present invention, when a predetermined forbidding condition is satisfied, transmission of data to the communication device or the monitoring device is refrained. Since data transmission to the monitoring device is not performed during when the forbidding condition is satisfied, the user is prevented from being charged unexpectedly high communication charge.

Other features and advantages of the present invention will become more apparent from detailed description given below with reference to the accompanying drawings.

### BRIEF DESCRIPTION OF THE DRAWINGS

Fig. 1 schematically illustrates a monitoring system using a blood glucose level measuring device according to a first embodiment of the present invention;
Fig. 2 is a block diagram of the structure of the blood glucose level measuring device according to the first embodiment;
Fig. 3 is a flowchart showing an automatic transmission process performed by the controller of the blood glucose level measuring device according to the first embodiment;
Fig. 4 is a block diagram of the structure of a blood glucose level measuring device according to a second embodiment;
Fig. 5 is a block diagram of the structure of a communication device according to a second embodiment;
Fig. 6 is a flowchart showing an automatic transmission process performed by the controller of the communication device according to the second embodiment;
Fig. 7 is a block diagram of the structure of the blood glucose level measuring device according to the third embodiment;
Fig. 8 is a flowchart showing an automatic transmission process performed by the controller of the blood glucose level measuring device according to the third embodiment; and
Fig. 9 schematically illustrates a monitoring system using a conventional blood glucose level measuring device.

### DETAILED DESCRIPTION OF THE PREFERRED EMBODIMENTS

Preferred embodiments of the present invention in an instance applied to a blood glucose level measuring device are described below with reference to the accompanying drawings.

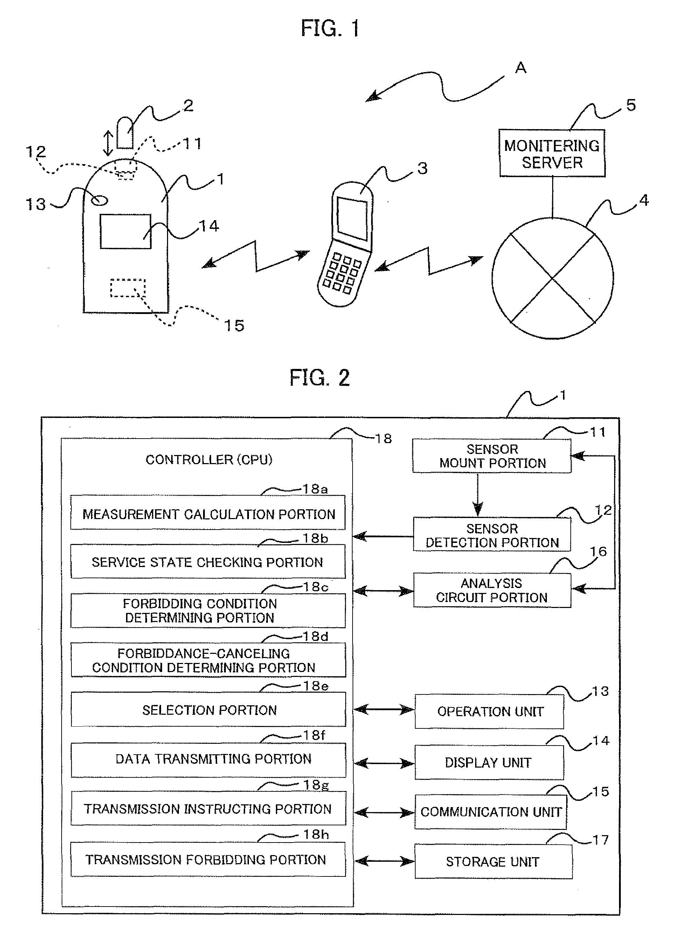

Fig. 1 schematically illustrates a monitoring system using a blood glucose level measuring device according to a first embodiment of the present invention. Fig. 2 is a block diagram of the blood glucose level measuring device according to the first embodiment.

The monitoring system A is a system for monitoring the blood glucose level of a patient. As shown in Fig. 1, the monitoring system A includes a blood glucose level measuring device 1, a communication device 3, a network 4 and a monitoring server 5. The patient measures the blood glucose level by using the blood glucose level measuring device 1. The blood glucose level measuring device 1 measures the blood glucose level in the blood applied to a sensor 2 mounted to the device and sends the blood glucose-related data to the communication device 3.

The communication device 3 is a device having a communication function and comprises e.g. a mobile phone. The communication device 3 sends the blood glucose-related data received from the blood glucose level measuring device 1 to the monitoring server 5 via the network. The communication device 3 always recognizes the radio base station tobe connected. When the patient is in a foreign country and the communication device 3 cannot be connected to the radio base station of the communication company with which the patient is under contract, communication using the international roaming service is available if the communication device 3 can be connected to a radio base station of an associated communication company. The communication device 3 is capable of sending to the blood glucose level measuring device 1 the information about the communication network corresponding to the radio base station to be connected.

The monitoring server 5 is a server for storing and monitoring the blood glucose-related data. The patient and the patient' s doctor can check the stored blood glucose-related data by making access to the monitoring server 5.

As shown in Fig. 2, the blood glucose level measuring device 1 includes a sensor mount portion 11, a sensor detection portion 12, an operation unit 13, a display unit 14, a communication unit 15, an analysis circuit portion 16, a storage unit 17 and a controller (CPU) 18. To measure the blood glucose level, a sensor 2 is mounted to the sensor mount portion 11 of the blood glucose level measuring device 1.

The sensor mount portion 11 is a portion where the sensor 2 is to be mounted, and comprises a recess formed at the front end (see Fig. 1) of the body of the blood glucose level measuring device 1 and designed to receive an end of the sensor 2. The structure or shape of the sensor mount portion 11 is not limited to this and may be designed in accordance with the shape or characteristics of the sensor 2. The sensor 2 includes a portion to which blood is to be applied, and electrodes for applying a voltage to the portion (neither shown). The sensor mount portion 11 is provided with electrodes (not shown) for connection to the electrodes of the sensor 2.

The sensor detection portion 12 serves to detect whether or not the sensor 2 is mounted to the sensor mount portion 11. The sensor mount portion 11A has a switch (not shown) in it. When the sensor 2 is inserted into the sensor mount portion 11, the switch is pushed by the sensor 2 and turned on. The sensor detection portion 12 detects that the switch is turned on and sends a signal indicating the mounting of the sensor 2 to the controller 18. Based on these signals from the sensor detection portion 12, the controller 18 determines whether or not the sensor 2 is mounted. The structure of the sensor detection portion 12 is not limited to this and may be designed in other ways as long as it properly detects the mounting of the sensor 2.

In this embodiment, the controller 18 turns on the power to allow measurement when the sensor 2 is mounted, and turns off the power when the sensor 2 is no more detected after the lapse of a predetermined time. In this embodiment, signals generated due to mounting and dismounting of the sensor 2 are utilized as information inputted for performing various settings. Specifically, removing the sensor 2 from the sensor mount portion 11 and subsequently inserting the sensor 2 again within a predetermined period of time (hereinafter referred to as "sensor removal/re-insertion") is utilized as an information input operation. This input operation by sensor removal/re-insertion can be used for selecting a storage mode, which will be described later, or setting a communication network, for example. The sensor removal/re-insertion may be used as different information inputting operations depending on the time interval between the removal and the re-insertion of the sensor 2. Further, performing the sensor removal/re-insertion twice may be used as another input operation.

The operation unit 13 includes a button which is pressed for inputting information. The button is arranged on the front surface of the body of the blood glucose level measuring device 1 (obverse side in the sheet surface of Fig. 1). When the button is pressed, the operation unit 13 inputs an operation signal into the controller 18. Due to the input of the signal, the controller 18 finds that information is input through the operation unit 13. The arrangement or number of the button is not limited to that shown in Fig. 1. When all the information is to be inputted by the sensor removal/re-insertion, the operation unit 13 may not be provided.

The display unit 14 is provided for displaying the measured blood glucose level, a warning message and so on. The display unit 14 is arranged on the front surface of the body of the blood glucose level measuring device 1 and may comprise e.g. a display screen of a liquid crystal display device.

The communication unit 15 is designed to perform short distance wireless communication using the Bluetooth (registered trademark) technology. Pairing between the communication unit 15 and the communication device 3 is performed in advance so that the communication unit can send and receive information to and from the communication device 3. The communication unit 15 is not limited to this and may be designed to perform communication using other techniques such as infrared communication. The communication with the communication device 3 is not limited to wireless communication, but may be performed by connecting the blood glucose level measuring device 1 to the communication device 3 by using a cable. However, the present invention is more effective for a system in which the blood glucose level measuring device 1 and the communication device 3 are to perform wireless communication, because in such a system the patient is less likely to notice the fact that the blood glucose level measuring device 1 and the communication device 3 are performing communication and that the blood glucose-related data are being sent.

The analysis circuit portion 16 is provided for applying a voltage to the electrodes of the sensor 2 via the electrodes of the sensor mount portion 11 when blood is applied to a predetermined portion of the sensor 2 mounted to the sensor mount portion 11 and causes generation of a responsive current corresponding to the blood glucose level in the blood. Further, the analysis circuit portion 16 converts the responsive current into a voltage and inputs the voltage signal into the controller 18. The measurement calculation portion 18a (which will be described later) of the controller 18 calculates the glucose concentration based on the inputted voltage signal. The glucose concentration represents the blood glucose level. Since this method for measuring the blood glucose level is known, the detailed description is omitted. The structure including the sensor 2, the sensor mount portion 11, the analysis circuit portion 16 and the controller 18 (the measurement calculation portion 18a) corresponds to the "measurer" of the present invention. The measurer measures the blood glucose level. The method for measuring the blood glucose level is not limited to this, and the blood glucose level may be measured by other methods.

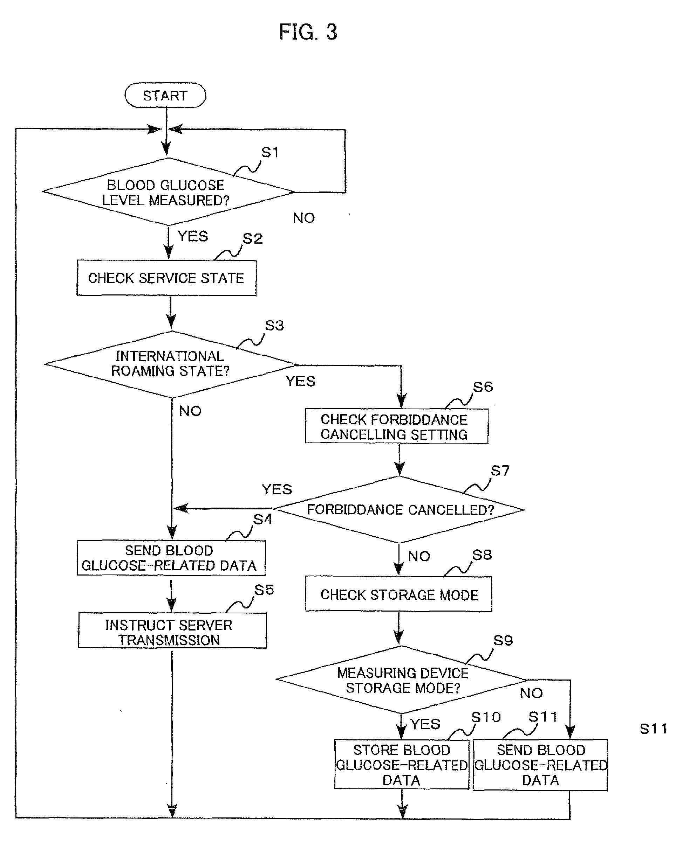

The storage unit 17 includes e.g. a ROM, a RAM and a nonvolatile memory. The ROM stores e.g. a control program executed by the controller 18. The RAM provides an area for temporarily storing data such as blood glucose-related data and a work area used by the controller 18 for e.g. computation. The nonvolatile memory stores e.g. blood glucose-related data. It is to be noted that the program for automatic transmission process shown in the flowchart of Fig. 3, which will be described later, may be stored in the ROM in advance or may be downloaded via the network 4 and the communication device 3 and stored in the nonvolatile memory.

The controller 18 performs various kinds of control and signal processing of the blood glucose level measuring device 1 and comprises e.g. a CPU. From the functional point of view, the controller 18 includes the measurement calculation portion 18a, a service state checking portion 18b, a forbidding condition determining portion 18c, a forbiddance-cancelling condition determining portion 18d, a selection portion 18e, a data transmitting portion 18f, a transmission instructing portion 18g and a transmission forbidding portion 18h.

The measurement calculation portion 18a serves to calculate the blood glucose level based on a voltage signal sent from the analysis circuit portion 16. The data indicating the date and time of the measurement and the identification data for identifying the patient are added to the data of the blood glucose level calculated by the measurement calculation portion 18a. The data obtained in this way are the blood glucose-related data.

The service state checking portion 18b serves to check the service state of the communication device 3. By the service state checking portion 18b, the service state is checked, including whether or not the communication device 3 is in a condition to use the international roaming service for communication. (Hereinafter this condition is referred to as "international roaming state".) In the international roaming state, connection to a radio base station of the subscribed communication company is not possible, but connection to a radio base station of an associated foreign communication company is possible. Specifically, the service state checking portion 18b sends to the communication device 3 a signal for requesting information about the service state, via the communication unit 15. Upon receiving the signal, the communication device 3 checks the service state and sends to the communication unit 15 information about the service state, including the information on whether or not it is in the international roaming state. The service state checking portion 18b receives the responsive information via the communication unit 15.

The forbidding condition determining portion 18c serves to determine, based on the information about the service state which the service state checking portion 18b has received, whether the communication device 3 satisfies the condition for forbidding transmission of blood glucose-related data to the monitoring server 5. In this embodiment, the condition for forbidding blood glucose-related data transmission is that the communication device 3 is in the international roaming state. However, the forbidding condition is not limited to this. The forbidding condition and the way to determine whether or not the condition is satisfied can be set appropriately.

In this embodiment, a forbiddance-cancelling setting, which allows transmission of blood glucose-related data to the monitoring server 5 even when the forbidding condition is satisfied, can be made effective in advance. The forbiddance-cancelling condition determining portion 18d serves to determine whether or not this forbiddance-cancelling setting is made effective. That is, the forbiddance-cancelling condition determining portion 18d determines whether or not the forbiddance-cancelling condition that the forbiddance-cancelling setting is effective is satisfied. When this setting is made effective in advance, the forbiddance-cancelling condition determining portion 18d determines that the forbiddance-cancelling condition is satisfied. In this case, the blood glucose-related data are sent to the monitoring server 5 even when the forbiddance conditionissatisfied. This is effective when glucose-related data needs to be sent immediately to the monitoring server 5 regardless of the communication charge.

The forbiddance-cancelling condition is not limited to the above. For instance, the forbiddance of glucose-related data transmission may be cancelled when the area for storing glucose-related data in the storage unit is full in both the blood glucose level measuring device 1 and in the communication device 3. In this case, such a situation that glucose-related data are not saved or overwritten and erased can be avoided. The system may be arranged such that the patient can choose to send or not to send glucose-related data to the monitoring server 5 when the above-described forbidding condition is satisfied. Specifically, in this case, when the above-described forbidding condition is satisfied, a message to notify the patient to that effect and ask the patient if the immediate data transmission is really unnecessary may be displayed on the display unit 14. If the patient chooses to send the blood glucose-related data, it is determined that the forbiddance-cancelling condition is satisfied. According to this arrangement, whether to send or not to send glucose-related data to the monitoring server 5 is determined in accordance with the patient's intention. It is to be noted that the forbiddance-cancelling condition may not be set and the forbiddance-cancelling condition determining portion 18d may not be provided.

In this embodiment, in which storage unit the blood glucose-related data are to be stored when the blood glucose-related data are not to be sent to the monitoring server 5 can be selected and set in advance. That is, selection is possible among a measuring device storage mode for storing the blood glucose-related data in the storage unit 17 of the blood glucose level measuring device 1, a communication device storage mode for storing the blood glucose-related data in the storage unit of the communication device and a both-device storage mode for storing the blood glucose-related data in both the storage unit of the measuring device and the storage unit of the communication device. The selection portion 18e serves to select one of these storage modes. The selection portion 18e detects removal/re-insertion of the sensor based on a signal inputted from the sensor detection portion 12 and performs switching between the storage modes in response to the sensor removal/re-insertion. Specifically, when sensor removal/re-insertion is performed in the measuring device storage mode, the storage mode is switched from the measuring device storage mode to the communication device storage mode. When sensor removal/re-insertion is performed in the communication device storage mode, the storage mode is switched from the communication device storage mode to the both-device storage mode. When sensor removal/re-insertion is performed in the both-device storage mode, the storage mode is switched from the both-device storage mode to the measuring device storage mode. The method for selecting the storage mode is not limited to this. For instance, sensor removal/re-insertion and pressing of the button of the operation unit 13 may be used in combination for the selection. For instance, the display representing one of the storage modes on the display unit 14 is switched to another one by pressing the button of the operation unit 13, and the desired storage mode is set by performing sensor removal/re-insertion.

When the blood glucose level is calculated by the measurement calculation portion 18a, the data transmitting portion 18f serves to send the glucose-related data to the communication device 3 depending on the determination made by the transmission forbidding portion 18h and the selected storage mode. Since the communication unit 15 is wirelessly connected to the paired communication device 3, the data transmitting portion 18f sends glucose-related data to the communication device 3 by wireless communication. The transmission instructing portion 18g serves to send to the communication device 3 the transmission instruction information for instructing the communication device 3 to send glucose-related data to the monitoring server 5, depending on the determination made by the transmission forbidding portion 18h and the selected storage mode. The transmission instruction information includes information about the address of the monitoring server 5 and so on. Upon receiving the transmission instruction information, the communication device 3 sends the blood glucose-related data, received from the blood glucose level measuring device 1, to the monitoring server 5 via the network 4. The transmission instructing portion 18g does not need to be provided when the system is designed such that the communication device 3 automatically sends glucose-related data to the monitoring server 5 upon receiving the blood glucose-related data from the blood glucose level measuring device 1.

The transmission forbidding portion 18h serves to determine whether to send or not to send glucose-related data to the monitoring server 5, based on the determination made by the forbidding condition determining portion 18c and the forbiddance-cancelling condition determining portion 18d. When the forbidding condition determining portion 18c determines that the forbidding condition is satisfied and the forbiddance-cancelling condition determining portion 18d determines that the forbiddance-cancelling condition is not satisfied, the transmission forbidding portion 18h determines not to send glucose-related data to the monitoring server 5. On the other hand, when the forbidding condition determining portion 18c determines that the forbidding condition is not satisfied or when the forbidding condition determining portion 18c determines that the forbidding condition is satisfied but the forbiddance-cancelling condition determining portion 18d determines that the forbiddance-cancelling condition is satisfied, the transmission forbidding portion 18h determines to send glucose-related data to the monitoring server 5. In the case where the forbiddance-cancelling condition determining portion 18d is not provided, the transmission forbidding portion 18h determines not to send glucose-related data to the monitoring server 5 when the forbidding condition determining portion 18c determines that the forbidding condition is satisfied, and determines to send glucose-related data to the monitoring server 5 when the forbidding condition determining portion 18c determines that the forbidding condition is not satisfied.

When the transmission forbidding portion 18h determines to send glucose-related data to the monitoring server 5, the data transmitting portion 18f sends glucose-related data to the communication device 3, and the transmission instructing portion 18g sends transmission instruction information to the communication device 3. On the other hand, when the transmission forbidding portion 18h determines not to send glucose-related data to the monitoring server 5, transmission of the blood glucose-related data to the monitoring server 5 is refrained. In this case, each of the data transmitting portion 18f and the transmission instructing portion 18g performs processing corresponding to the selected storage mode.

Specifically, in the case of the measuring device storage mode, the data transmitting portion 18f does not send glucose-related data to the communication device 3 and the transmission instructing portion 18g does not send transmission instruction information to the communication device 3 so that the blood glucose-related data are stored in the storage unit 17. In the case of the communication device storage mode, the data transmitting portion 18f sends glucose-related data to the communication device 3, but the transmission instructing portion 18g does not send transmission instruction information to the communication device 3 so that the blood glucose-related data are stored in the storage unit of the communication device 3. In the both-device storage mode, the data transmitting portion 18f sends glucose-related data to the communication device 3, but the transmission instructing portion 18g does not send transmission instruction information to the communication device 3, and the blood glucose-related data are stored in the storage unit 17 and the storage unit of the communication device 3. The patient can change the storage unit for storing the blood glucose-related data by selecting the desired storage mode through e.g. sensor removal/re-insertion. Thus, for instance, when the remaining capacity of the storage area in the storage unit 17 for storing glucose-related data is small, the storage mode can be switched to the communication device storage mode, so that the blood glucose-related data are stored in the storage unit of the communication device 3. Alternatively, the system may be arranged such that the storage mode is automatically switched to the communication device storage mode when the remaining capacity of the storage area in the storage unit 17 for storing glucose-related data becomes small.

The blood glucose-related data stored in the storage unit 17 are collectively sent to the communication device 3 and then to the monitoring server 5 when the blood glucose level is measured and the transmission forbidding portion 18h determines to send the blood glucose-related data to the monitoring server 5. Also, the blood glucose-related data stored in the storage unit of the communication device 3 are collectively sent to the monitoring server 5 when the transmission forbidding portion 18h determines to send the blood glucose-related data to the monitoring server 5. In this way, the blood glucose-related data successively stored during when the forbidding condition is satisfied are collectively sent to the monitoring server 5 when the blood glucose level is measured in the state where the forbidding condition is no more satisfied. The system may be arranged such that the patient' s intention to send the blood glucose-related data is confirmed before the blood glucose-related data which have been stored are sent. The system may be arranged such that, when the situation changes so that the forbidding condition is no more satisfied, the blood glucose-related data which have been stored are sent to the monitoring server 5 without waiting for the next measurement of the blood glucose level.

The selection portion 18e may not be provided, and in which storage unit the glucose-related data are to be stored may be set in advance. For instance, the system may be designed such that, when the remaining capacity of the storage area in the storage unit 17 for storing glucose-related data is large, the data transmitting portion 18f does not send glucose-related data to the communication device 3 so that the blood glucose-related data are stored in the storage unit 17. The systemmay also be designed such that, when the remaining capacity of the storage area in the storage unit 17 for storing glucose-related data is small, the data transmitting portion 18f sends the blood glucose-related data to the transmission device 3 but the transmission instructing portion 18g does not send transmission instruction information to the communication device 3 so that the blood glucose-related data are stored in the storage unit of the communication device 3

The communication device 3 always recognizes the radio base station to be connected and can send to the blood glucose level measuring device 1 information about a communication network corresponding to the radio base station. In the case where a communication network setting mode for setting a communication network is selected, the controller 18 displays information representing the detected communication network on the display unit 14. When a plurality of communication networks are detected, the controller 18 displays a plurality of pieces of information each representing a respective one of the detected communication networks periodically one after another on the display unit 14. The controller 18 detects removal/re-insertion of the sensor based on a signal inputted from the sensor detection portion 12 and sets one of the detected communication networks as the network for performing communication. Specifically, the communication network which has been displayed on the display unit 14 when the sensor removal/re-insertion is performed is set as the communication network for performing communication with the monitoring server 5. The method of setting the communication network is not limitedtothis. For instance, the sensor removal/re-insertion and pressing of the button of the operation unit 13 may be used in combination for setting the communication network. In this case, for instance, the information representing one of the communication networks on the display unit 14 may be switched to another when the button of the operation unit 13 is pressed, and the desired communication network is set by performing sensor removal/re-insertion.

Fig. 3 is a flowchart showing an automatic transmission process performed by the controller 18. The automatic transmission process is a process for automatically sending blood glucose-related data to the monitoring server 5 when the blood glucose level is measured by a patient. The automatic transmission process starts when the power of the blood glucose level measuring device 1 is turned on and continues until the power of the blood glucose level measuring device 1 is turned off.

First, whether or not the blood glucose level has been measured is determined in Step S1. Specifically, this determination is made based on whether or not the measurement calculation portion 18a has calculated the blood glucose level. When it is determined that the blood glucose level has not been measured (if No in Step S1), the process returns to Step S1, and the checking is repeated until the blood glucose level is measured. When it is determined that the blood glucose level has been measured (if Yes in Step S1), the service state is checked in Step S2. Specifically, the service state checking portion 18b sends to the communication device 3 a signal for requesting information about the service state, and receives the information about the service state as a response.

Then, whether or not the communication device 3 is in the international roaming state is determined in Step S3. Specifically, in this step, the forbidding condition determining portion 18c determines whether or not the information indicating that the communication device 3 is in the international roaming state is included in the obtained information about the service state. When the communication device 3 is not in the international roaming state (if No in Step S3), blood glucose-related data are sent to the communication device 3 in Step S4. Then, in Step S5, the communication device 3 is instructed to send the blood glucose-related data to the monitoring server 5, and thereafter the process returns to Step S1. Specifically, in Step S5, the transmission instructing portion 18g sends transmission instruction information to the communication device 3 to instruct the communication device 3 to send glucose-related data to the monitoring server 5. Upon receiving the transmission instruction information, the communication device 3 sends the blood glucose-related data, received from the blood glucose level measuring device 1, to the monitoring server 5 by using information about the address of the monitoring server 5 included in the transmission instruction information. In this way, when the blood glucose level is measured, the blood glucose-related data are automatically sent to the monitoring server 5.

If it is determined in Step S3 that the communication device 3 is in the international roaming state (if Yes in Step S3), the state of the forbiddance cancelling setting is checked in Step S6, and whether or not the forbiddance is to be cancelled is determined in Step S7. Specifically, the forbiddance-cancelling condition determining portion 18d determines whether or not the forbiddance-cancelling setting is made effective. If the forbiddance of data transmission is cancelled (if Yes in Step S7), the blood glucose-related data are sent to the communication device 3 in Step S4 and also transmission instruction information is sent to the communication device 3 in Step S5, and thereafter, the process returns to Step S1. If the forbiddance of data transmission is not cancelled (if No in Step S7), the storage mode is checked in Step S8, and whether or not the storage mode is the measuring device storage mode is determined in Step S9.

If the storage mode is the measuring device storage mode (if Yes in Step S9), the blood glucose-related data are stored in the storage unit 17 in Step S10, and the process returns to Step S1. In this case, the blood glucose-related data are not sent to the communication device 3, and data transmission to the monitoring server 5 is not instructed to the communication device 3. On the other hand, if the storage mode is a mode other than the measuring device storage mode (if No in Step 59), the blood glucose-related data are sent to the communication device 3 in Step 511, and the process returns to Step S1. In this case again, data transmission to the monitoring server 5 is not instructed to the communication device 3. Thus, the communication device 3 does not send the received blood glucose-related data to the monitoring server 5 but stores the blood glucose-related data in its storage unit. In the both-device storage mode, the blood glucose-related data are stored not only in the storage unit of the communication device 3 but also in the storage unit 17.

According to this embodiment, in the case where the measuring device 3 is in the international roaming state when the blood glucose level is measured, transmission of the blood glucose-related data to the monitoring server 5 is refrained. This prevents the patient from being charged an extremely high communication charge as a result of performing data transmission to the monitoring server 5 in the international roaming state without recognizing it.

Moreover, when the forbiddance cancelling setting is made effective, the blood glucose-related data are sent to the monitoring server 5 even when the communication device 3 is in the international roaming state. Thus, when the patient does not care about the communication charge or immediate transmission of blood glucose-related data is necessary, the forbiddance cancelling setting can be made effective to arrange the system such that the blood glucose-related data are sent automatically to the monitoring server 5 when the blood glucose level is measured. Moreover, by setting the storage mode, the blood glucose-related data are stored in a desired storage unit when transmission of the blood glucose-related data to the monitoring server 5 is refrained. Since the blood glucose level measuring device 1 in this embodiment has the structure for forbidding data transmission, data transmission to the monitoring server 5 can be forbidden regardless of the kind of the communication device 3.

Although the fact that the communication device 3 is in the international roaming state is set as the forbidding condition in the first embodiment, the present invention is not limited to this. For instance, the fact that the time is not within a discount time period may be set as the forbidding condition. In this case, the forbidding condition determining portion 18c determines that the forbidding condition is satisfied when a contract for a billing plan which includes discount for communication within a predetermined time period is included in the service state and the time found by the clock function of the controller 18 is not within the discount time period. In this case, communication at any time outside the discount time period is forbidden, and communication is performed only within the discount time period. Thus, even if the patient measures the blood glucose level without paying attention to the time, the patient is prevented frombeing charged a communication charge to which discount is not applied. Similarly, in the case of a contract for a billing plan which sets fixed charge for communication within a predetermined time period, the fact that the time is not within the predetermined time period can be set as the forbidding condition.

Further, the fact that the communication is not within the range covered by a contract for a flat rate billing plan may be set as the forbidding condition. In this case, the forbidding condition determining portion 18c determines that the forbidding condition is satisfied when a contract for a flat rate billing plan is included in the service state and the flat rate charging will not be applied to the communication. In this instance, communication outside the range covered by the flat rate billing plan is forbidden, and only the communication within the range of the flat rate billing plan is allowed. Thus, even if the patient measures the blood glucose level without paying attention to the coverage of the flat rate billing plan, the patient is prevented from being charged an extra charge for the communication outside the coverage of the flat rate billing plan.

In some communication billing plans, the communication charge is calculated based on a measured rate system until the charge reaches a predetermined amount, and a flat rate system is applied after the predetermined amount is reached. The fact that a contract for this type of billing plan is included in the service state may be set as the forbidding condition, and the fact that the predetermined amount has been reached may be set as the forbiddance-cancelling condition. This allows the patient to save the communication charge, because communication is forbidden in the case where the predetermined amount has not been reached and allowed only in the case where the predetermined amount has been reached.

Although the first embodiment is described as to the instance where the measuring device is a blood glucose level measuring device 1 for measuring a blood glucose level and the monitoring system A is a system for monitoring the blood glucose level of a patient, the present invention is not limited to this. For instance, the system of the present invention may be designed as a system for monitoring a blood pressure by replacing the structure for measuring the blood glucose level in the blood glucose level measuring device 1 (i.e., the sensor 2, the sensor mount portion 11, the analysis circuit portion 16 and the measurement calculation portion 18a) with a structure for measuring a blood pressure. Similarly, the blood glucose level measuring device 1 may be replaced with a blood analyzer, a urine analyzer, an electrocardiogram monitor and so on to design the system of the present invention as a system for monitoring measurements of these devices. Moreover, the present invention is applicable not only to a medical monitoring system but also to a system that employs e.g. a sugar content meter or a position measuring device as a measuring device, instead of the blood glucose level measuring device 1.

Although the first embodiment is described as to the instance where the communication device 3 is a mobile phone, the present invention is not limited to this. The communication device 3 may be any kind of communication devices which can be carried, and the examples include a smartphone, a mobile information terminal and a laptop personal computer. The communication device 3 may be a fixed type (not for carriage) communication device such as a desktop personal computer or a fixed phone. Although such a fixed type communication device does not have a problem related to international roaming, the present invention is still effective when the patient is under a contract for a billing plan which includes discount for communication within a predetermined time period or for a flat rate billing plan or the like.

Although the first embodiment is described as to the instance where the blood glucose-related data and the transmission instruction information are sent separately to the communication device 3, the present invention is not limited to this arrangement. Namely, the transmission instruction information may be included in the blood glucose-related data. In this case, when the storage mode is a mode other than the measuring device storage mode, the blood glucose-related data are sent to the communication device 3, with the transmission instruction information deleted from the blood glucose-related data.

Although the first embodiment is described as to the instance where the structure for forbidding transmission is provided in the blood glucose level measuring device 1, the present invention is not limited to this arrangement. For instance, the structure for forbidding transmission may be provided in the communication device 3. This arrangement is described below as a second embodiment.

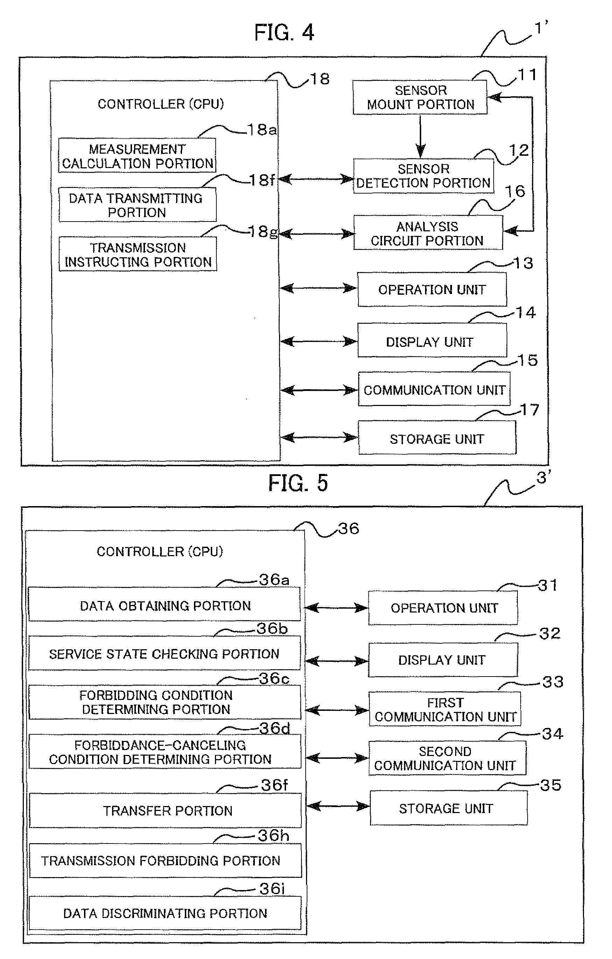

Fig. 4 is a block diagram of the structure of a blood glucose level measuring device according to the second embodiment. In this figure, the elements that are identical or similar to those of the blood glucose level measuring device 1 shown in Fig. 2 are designated by the same reference signs as those used for the blood glucose level measuring device 1.

The blood glucose level measuring device 1' of this embodiment differs from the blood glucose level measuring device 1 only in that it does not include the service state checking portion 18b, the forbidding condition determining portion 18c, the forbiddance-cancelling condition determining portion 18d, the selection portion 18e and the transmission forbidding portion 18h.

The blood glucose level measuring device 1' does not include the structure for forbidding data transmission. Thus, when the blood glucose level is calculated by the measurement calculationportion 18a, the data transmittingportion 18f sends the blood glucose-related data to the communication device 3', and the transmission instructing portion 18g sends transmission instruction information to the communication device 3'. Other structures of the blood glucose level measuring device 1' are the same as those of the blood glucose level measuring device 1, the description is omitted.

Fig. 5 is a block diagram of the structure of a communication device according to the second embodiment.

The communication device 3' of this embodiment includes an operation unit 31, a display unit 32, a first communication unit 33, a second communication unit 34, a storage unit 35 and a controller (CPU) 36. The structure of the communication device 3' is the same as that of a typical mobile phone, except that the functional structure of the controller 36 includes a portion for forbidding data transmission.

The operation unit 31 includes a plurality of buttons which are pressed for inputting information. The arrangement and function of the buttons are the same as those of a typical mobile phone. When any of the buttons is pressed, the operation unit 31 inputs an operation signal corresponding to the pressedbutton into the controller 36. The controller 36 finds out which one of the buttons is pressed based on the inputted operation signal and performs the appropriate processing.

The display unit 32 is provided for displaying e.g. telephone numbers or various messages. The display unit 32 may comprise e.g. a display screen of a liquid crystal display device.

The first communication unit 33 is designed to perform short distance wireless communication using the Bluetooth (registered trademark) technology. Pairing between the first communication unit 33 and the blood glucose level measuring device 1' is performed in advance so that the first communication unit 33 can send and receive information to and from the blood glucose level measuring device 1'. The first communication unit 33 is not limited to this and may be designed to perform communication using other techniques such as infrared communication. The communication with the blood glucose level measuring device 1' is not limited to wireless communication, but may be performed by connecting the blood glucose level measuring device 1' to the communication device 3' by using a cable.

The second communication unit 34 is designed to perform communication with the monitoring server 5 via the network 4. Specifically, the second communication unit 34 performs wireless communication with a radio base station, not shown, and is connected to the network 4 connected to the radio base station.

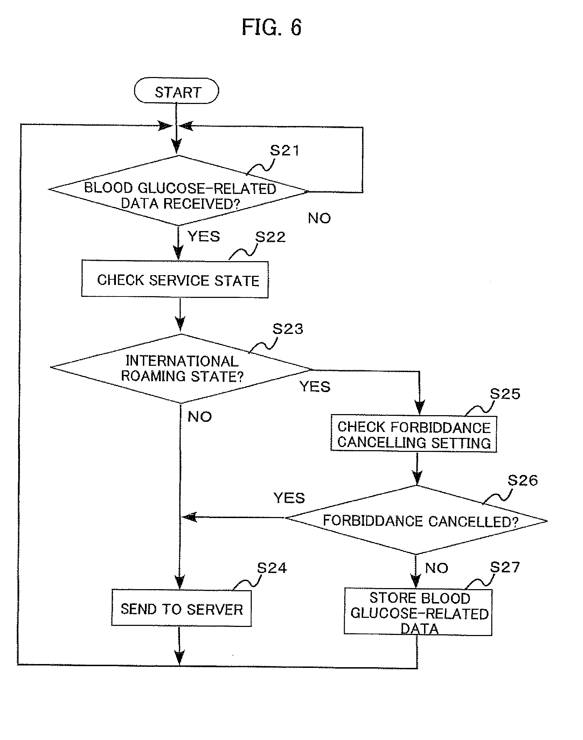

The storage unit 35 includes e.g. a ROM, a RAM and a nonvolatile memory. The ROM stores e.g. a control program executed by the controller 36. The RAM provides an area for temporarily storing information and a work area used by the controller 36 for e.g. computation. The nonvolatile memory stores e.g. blood glucose-related data. It is to be noted that the program for automatic transmission process shown in the flowchart of Fig. 6, which will be described later, may be stored in the ROM in advance or may be downloaded via the network 4 and stored in the nonvolatile memory.

The controller 36 performs various kinds of control and signal processing of the communication device 3' and comprises e.g. a CPU. From the functional point of view, the controller 36 includes a data obtainingportion 36a, a service state checking portion 36b, a forbidding condition determining portion 36c, a forbiddance-cancelling condition determining portion 36d, a transfer portion 36f, a transmission forbidding portion 36h, and a data discriminating portion 36i.

The data obtaining portion 36a serves to obtain the data sent from the blood glucose level measuring device 1' via the first communication unit 33. Since the first communication unit 33 is wirelessly connected to the paired blood glucose level measuring device 1', the data obtaining portion 36a obtains glucose-related data from the blood glucose level measuring device 1' by wireless communication. The data obtaining portion 36a can obtain data other than blood glucose-related data as well. For instance, the data obtaining portion 36a can obtain data on a blood pressure obtained by a blood pressure measuring device, not shown.

The data discriminating portion 36i discriminates among a plurality of kinds of data obtained by the data obtaining portion 36a. The data discriminating portion 36i recognizes blood glucose-related data when information indicating it is included in the obtained data.

The service state checking portion 36b serves to check the service state of the communication device 3'. By the service state checking portion 36b, the service state is checked, including whether or not the communication device 3' is in a condition to use the international roaming service for communication. The communication device 3' is determined to be in the international roaming state when connection to a radio base station of the subscribed communication company is not possible and connection to a radio base station of an associated foreign communication company is possible.

Based on the information about the service state which the service state checking portion 36b has received, the forbidding condition determining portion 36c determines whether or not the forbidding condition is satisfied, i.e., the communication device 3'is in the international roaming state. However, the forbidding condition is not limited to this. The forbidding condition and the way to determine whether or not the condition is satisfied can be set appropriately.

In this embodiment, a forbiddance-cancelling setting, which allows transmission of blood glucose-related data to the monitoring server 5 even when the forbidding condition is satisfied, can be made effective in advance. The forbiddance-cancelling condition determining portion 36d serves to determine whether or not this forbiddance-cancelling setting is made effective. That is, the forbiddance-cancelling condition determining portion 36d determines whether or not the forbiddance-cancelling condition that the forbiddance-cancelling setting is effective is satisfied. When this setting is made effective in advance, the forbiddance-cancelling condition determining portion 36d determines that the forbiddance-cancelling condition is satisfied. In this case, the blood glucose-related data are sent to the monitoring server 5 even when the forbiddance conditionissatisfied. This is effective when glucose-related data needs to be sent immediately to the monitoring server 5 regardless of the communication charge.

The forbiddance-cancelling condition is not limited to the above. For instance, the forbiddance of glucose-related data transmission may be cancelled when the area for storing glucose-related data in the storage unit 35 is full. Alternatively, when the above-described forbidding condition is satisfied, a message to notify the patient to that effect and ask the patient if the immediate data transmission is really unnecessary may be displayed on the display unit 32, to allow the patient to choose to send or not to send the blood glucose-related data. It is to be noted that the forbiddance-cancelling condition may not be set and the forbiddance-cancelling condition determining portion 18d may not be provided.

When the data obtaining portion 36a obtains blood glucose-related data and the controller 36 receives the transmission instruction information from the blood glucose level measuring device 1', the transfer portion 36f transfers the blood glucose-related data to the monitoring server 5 via the second communication unit 34 and the network 4, in accordance with the determination by the transmission forbidding portion 36h. The transfer portion 36f can also send data other than the blood glucose-related data to corresponding servers. Information about the address of each server is stored in the storage unit 35 in advance, and each kind of data discriminated by the data discriminating portion 36i is sent to the corresponding server. Alternatively, information about the address of the monitoring server 5 may be included in the transmission instruction information, so that the data transmission to the monitoring server 5 can be performed by utilizing this information. The transfer portion 36f may be arranged to automatically send the blood glucose-related data to the monitoring server 5 when the data obtaining portion 36a obtains the blood glucose-related data. In this case, the blood glucose level measuring device 1' does not need to send the transmission instruction information.

The transmission forbidding portion 36h serves to determine whether to send or not to send glucose-related data to the monitoring server 5, based on the determination made by the forbidding condition determining portion 36c and the forbiddance-cancelling condition determining portion 36d. When the forbidding condition determining portion 36c determines that the forbidding condition is satisfied and the forbiddance-cancelling condition determining portion 36d determines that the forbiddance-cancelling condition is not satisfied, the transmission forbidding portion 36h determines not to send glucose-related data to the monitoring server 5. On the other hand, when the forbidding condition determining portion 36c determines that the forbidding condition is not satisfied or when the forbidding condition determining portion 36c determines that the forbidding condition is satisfied but the forbiddance-cancelling condition determining portion 36d determines that the forbiddance-cancelling condition is satisfied, the transmission forbidding portion 36h determines to send glucose-related data to the monitoring server 5. In the case where the forbiddance-cancelling condition determining portion 36d is not provided, the transmission forbidding portion 36h determines not to send glucose-related data to the monitoring server 5 when the forbidding condition determining portion 36c determines that the forbidding condition is satisfied, and determines to send glucose-related data to the monitoring server 5 when the forbidding condition determining portion 36c determines that the forbidding condition is not satisfied.

When the transmission forbidding portion 36h determines to send glucose-related data to the monitoring server 5, the transfer portion 36f sends glucose-related data to the monitoring server 5. On the other hand, when the transmission forbidding portion 36h determines not to send glucose-related data to the monitoring server 5, transmission of the blood glucose-related data to the monitoring server 5 is refrained. In this case, the transfer portion 36f does not send the blood glucose-related data to the monitoring server 5, and the blood glucose-related data are stored in the storage unit 35.

The blood glucose-related data stored in the storage unit 35 are collectively sent to the monitoring server 5 when blood glucose-related data are obtained and the transmission forbidding portion 36h determines to send the blood glucose-related data to the monitoring server 5. In this way, the blood glucose-related data successively stored during when the forbidding condition is satisfied are collectively sent to the monitoring server 5 when blood glucose-related data are obtained in the state where the forbidding condition is no more satisfied. The system may be arranged such that the patient's intention to send the blood glucose-related data is confirmed before the blood glucose-related data which have been stored are sent. The system may be arranged such that, when the situation changes so that the forbidding condition is no more satisfied, the blood glucose-related data which have been stored are sent to the monitoring server 5 without waiting for the next obtainment of the blood glucose-related data.

Fig. 6 is a flowchart showing an automatic transmission process performed by the controller 36. The automatic transmission process is a process for automatically sending to the monitoring server 5 the blood glucose-related data which the communication device 3' has received from the blood glucose level measuring device 1'. The automatic transmission process starts when the power of the communication device 3' is turned on and continues until the power of the communication device 3' is turned off.

First, whether or not the blood glucose-related data and transmission instruction information have been received is determined in Step S21. When it is determined that the blood glucose-related data and transmission instruction information have not been received (if No in Step S21), the process returns to Step 521, and the checking is repeated until these are received. When it is determined that these have been received (if Yes in Step S21), the service state is checked in Step S22.

Then, whether or not the communication device 3' is in the international roaming state is determined in Step S23. Specifically, in this step, the forbidding condition determining portion 36c determines whether or not the information indicating that the communication device 3' is in the international roaming state is included in the obtained information about the service state. When the communication device 3' is not in the international roaming state (if No in Step S23), the transfer portion 36f sends the blood glucose-related data to the monitoring device 5 in Step S24, and thereafter the process returns to Step 521. Specifically, in Step S24, the communication device 3', which has received the blood glucose-related data and the transmission instruction information, sends the blood glucose-related data to the monitoring server 5 by using information about the address of the monitoring server 5 stored in the storage unit 35. In this way, when the blood glucose level is measured by the blood glucose level measuring device 1', the blood glucose-related data are automatically sent to the monitoring server 5.

If it is determined in Step S23 that the communication device 3' is in the international roaming state (if Yes in Step 523), the state of the forbiddance cancelling setting is checked in Step S25, and whether or not the forbiddance is to be cancelled is determined in Step S26. If the forbiddance of data transmission is cancelled (if Yes in Step S26), the blood glucose-related data are sent to the monitoring server 5 in Step S24, and thereafter, the process returns to Step S21. If the forbiddance of data transmission is not cancelled (if No in Step S26), the blood glucose-related data are stored in the storage unit 35 in Step S27, and the process returns to Step S21. In this case, the blood glucose-related data are not sent to the monitoring server 5.

The blood glucose level measuring device 1' according to the second embodiment does not have a structure for forbidding data transmission. However, the communication device 3' according to the second embodiment has the structure for forbidding data transmission similar to that of the blood glucose level measuring device 1 according to the first embodiment. Thus, the second embodiment provides the same advantages as those of the first embodiment. Further, in the second embodiment, the communication device 3' is capable of sending a plurality of kinds of data to corresponding servers. Thus, it is possible to send each kind of data transmitted from different measuring devices to a corresponding server.

Although the fact that the communication device 3' is in the international roaming state is set as the forbidding condition in the second embodiment, the present invention is not limited to this. Further, although description is made as to the instance where the measuring device 1' is a device for measuring the blood glucose level, the monitoring system A is a system for monitoring the blood glucose level of a patient, and the communication device 3' is a mobile phone, the present invention is not limited to this. Similarly to the first embodiment, the forbidding condition can be changed, the blood glucose level measuring device 1' may be replaced with other kinds of measuring devices, and the communication device 3' may be a device other than a mobile phone.

Although the second embodiment is described as to the instance where the blood glucose-related data and the transmission instruction information are sent separately to the communication device 3', the present invention is not limited to this arrangement. Namely, the transmission instruction information may be included in the blood glucose-related data.

According to the second embodiment, the data which the communication device 3' has received from the blood glucose level measuring device 1' are sent as they are to the monitoring server 5. However, the present invention is not limited to this. For instance, the data which the communication device 3' has received from the blood glucose level measuring device 1' may be processed before being sent to the monitoring server 5. The communication device 3' may receive data from a device other than a measuring device or the communication device 3' may obtain data by performing data processing inside the communication device 3'. For instance, the present invention is applicable to the instance in which e-mails prepared using a function of the communication device 3' are sent to the monitoring server 5.

The blood glucose level measuring device 1 according to the first embodiment may be arranged to have a communication function. The blood glucose level measuring device having a communication function is describedbelow as a third embodiment. In this case, the monitoring system A (see Fig. 1) does not include a communication device 3.

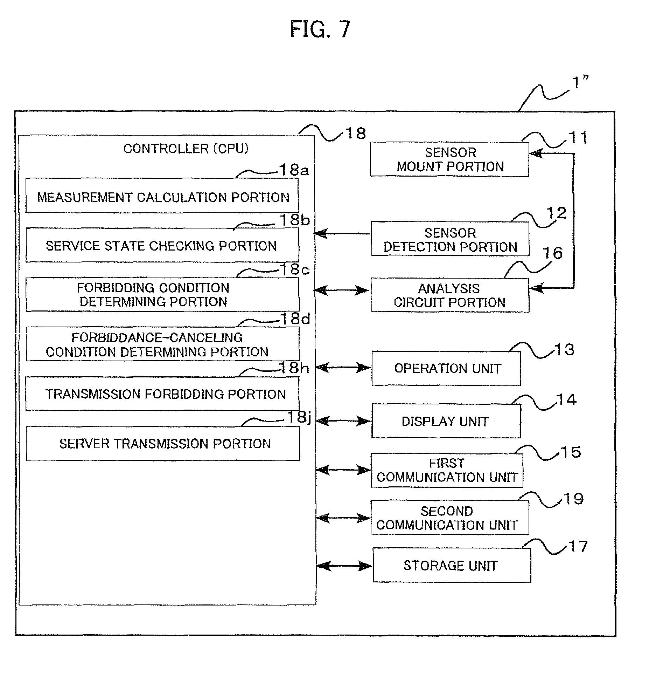

Fig. 7 is a block diagram of the structure of a blood glucose level measuring device 1" according to the third embodiment. In this figure, the elements that are identical or similar to those of the blood glucose level measuring device 1 shown in Fig. 2 are designated by the same reference signs as those used for the blood glucose level measuring device 1.

The blood glucose level measuring device 1" differs from the blood glucose level measuring device 1 of the first embodiment in that the blood glucose level measuring device 1" does not include the selection portion 18e, the data transmitting portion 18f and the transmission instructing portion 18g, but includes a server transmission portion 18j and a second communication unit 19. The first communication unit 15 corresponds to the communication unit 15 of the blood glucose level measuring device 1. The server transmission portion 18j and the second communication unit 19 correspond,respectively,to the transfer portion 36f and the second communication unit 34 of the communication device 3' according to the second embodiment. However, the server transmission portion 18j differs from the transfer portion 36f in that the server transmission portion 18j sends blood glucose-related data to the monitoring server 5 when the blood glucose level is calculated at the measurement calculation portion 18a.

Since the blood glucose level measuring device 1" does not need to send data to another communication device, the blood glucose level measuring device 1" does not include the data transmitting portion 18f and the transmission instructing portion 18g. On the other hand, the blood glucose level measuring device 1" needs to communicate with the monitoring server 5 via the network 4, so that the blood glucose level measuring device 1" includes the server transmission portion 18j and the second communication unit 19. Since the blood glucose level measuring device 1" does not need to store blood glucose-related data in a storage unit of another communication device, the blood glucose level measuring device 1" does not include the selection portion 18e. Since the function and structure of each structural element are the same as those of a corresponding element, its detailed description is omitted.

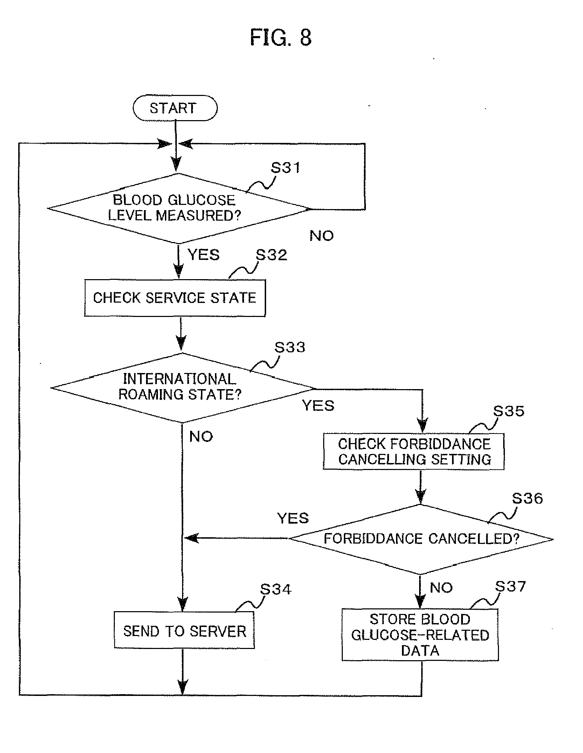

Fig. 8 is a flowchart showing an automatic transmission process performed by the controller 18. The automatic transmission process is a process for automatically sending to the monitoring server 5 the blood glucose-related data when the blood glucose level is measured by a patient. The automatic transmission process starts when the power of the blood glucose level measuring device 1" is turned on and continues until the power of the blood glucose level measuring device 1" is turned off.

First, whether or not the blood glucose level has been measured is determined in Step S31. When it is determined that the blood glucose level has not been measured (if No in Step S31), the process returns to Step S31, and the checking is repeated until the blood glucose level is measured. When it is determined that the blood glucose level has been measured (if Yes in Step S31), the service state checking portion 18b checks the service state in Step S32.

Then, the forbidding condition determining portion 18c determines in Step S33 whether or not the blood glucose level measuring device 1" is in the international roaming state. When the blood glucose level measuring device 1" is not in the international roaming state (if No in Step S33), the server transmission portion 18j sends the blood glucose-related data to the monitoring server 5 in Step S34, and the process returns to Step S31. In this way, when the blood glucose level is measured, the blood glucose-related data are automatically sent to the monitoring server 5.

If it is determined in Step S33 that the blood glucose level measuring device 1" is in the international roaming state (if Yes in Step S33), the state of the forbiddance cancelling setting is checked in Step 535, and the forbiddance-cancelling condition determining portion 18d determines in Step S36 whether or not the forbiddance is to be cancelled. If the forbiddance of data transmission is cancelled (if Yes in Step S36), the blood glucose-related data are sent to the monitoring server 5 in Step S34, and the process returns to Step S31. If the forbiddance of data transmission is not cancelled (if No in Step S36), the blood glucose-related data are stored in the storage unit 17 in Step S37, and the process returns to Step S31. In this case, the blood glucose-related data are not sent to the monitoring server 5.

The blood glucose level measuring device 1" according to the third embodiment has the structure for forbidding data transmission. Thus, the third embodiment provides the same advantages as those of the first embodiment. Further, the blood glucose level measuring device 1" of the third embodiment has the structure for communicating with the monitoring server 5. Thus, a separate communication device for performing communication with the monitoring server 5 is not necessary.

Although the fact that the blood glucose level measuring device 1" is in the international roaming state is set as the forbidding condition in the third embodiment, the present invention is not limited to this. Further, although description is made as to the instance where the measuring device 1' is a device for measuring the blood glucose level and the monitoring system A is a system for monitoring the blood glucose level of a patient, the present invention is not limited to this. Similarly to the first embodiment, the forbidding condition can be changed, and the measuring device may be a device other than a blood glucose level measuring device.

The measuring device, the communication device, the monitoring system and the program according to the present invention are not limited to the above-described embodiments. The specific structure of each part of the measuring device, communication device, monitoring system and program may be varied in design in many ways.

(Appendix 1)A measuring device in a monitoring system including a monitoring device for monitoring data received via a network, the measuring device comprising:
a measurer for measuring the data;
a monitoring-device transmitter for sending the data measured by the measurer to the monitoring device;
a forbidding condition determiner for determining whether or not a predetermined forbidding condition is satisfied; and
a transmission forbidder for forbidding the monitoring-device transmitter from sending the data to the monitoring device when the forbidding condition determiner determines that the predetermined forbidding condition is satisfied.

(Appendix 2) The measuring device according to Appendix 1, further comprising a service state checker for checking a communication service state,
wherein the forbidding condition determiner determines that the predetermined forbidding condition is satisfied when the service state checker finds that a predetermined service is to be performed.

## Claims

1. A measuring device in a monitoring system including a monitoring device for monitoring data received via a network, the measuring device comprising:
a measurer for measuring the data;
a data transmitter for sending the data to a communication device that performs communication with the monitoring device;
a forbidding condition determiner for determining whether or not a predetermined forbidding condition is satisfied; and
a transmission forbidder for forbidding transmission of the data to the communication device or to the monitoring device when the forbidding condition determiner determines that the predetermined forbidding condition is satisfied.

2. The measuring device according to claim 1, wherein the communication device includes a first storage for storing the data sent by the data transmitter, and the measuring device further comprises:
a second storage for storing the data measured by the measurer;
a selector for selecting to store the data in the first storage only, in the second storage only, or in both the first storage and the second storage; and
a transmission instructor for instructing the communication device to send the data to the monitoring device;
wherein the transmission forbidder forbids the transmission instructor from instructing to send the data when storing the data in the first storage is set by the selector; and
wherein the transmission forbidder forbids the data transmitter from sending the data when storing the data in the second storage only is set by the selector.

3. The measuring device according to claim 2, further comprising:
a sensor mount portion for mounting a sensor to which a measurement object is to be applied; and
a sensor detector for detecting mounting and dismounting of the sensor to and from the sensor mount portion;
wherein the selector performs a selection operation in accordance with mounting or dismounting of the sensor detected by the sensor detector.

4. The measuring device according to claim 2 or 3, further comprising a service state checker for checking a communication service state of the communication device,
wherein the forbidding condition determiner determines that the predetermined forbidding condition is satisfied when the service state checker finds that a predetermined service is to be performed.

5. The measuring device according to claim 4, wherein the predetermined service is international roaming.

6. The measuring device according to any one of claims 2-5, wherein the transmission instructor sends to the communication device an address of the monitoring device as well in instructing the communication device to send the data.

7. The measuring device according to any one of claims 1-6, wherein the communication device includes a network detector for detecting communication networks available for performing communication with the monitoring device, and the measuring device further comprises:
a network information receiver for receiving a plurality of pieces of network information each representing a respective one of the communication networks detected by the network detector;
a display unit for displaying the pieces of network information;
a network selector for selecting one from the pieces of network information displayed on the display unit;
a network setter for setting the communication network represented by the piece of information selected by the network selector as a communication network for the communication device to perform communication with the monitoring device;
a sensor mount portion for mounting a sensor to which a measurement object is to be applied; and
a sensor detector for detecting mounting and dismounting of the sensor to and from the sensor mount portion;
wherein the network selector performs a network selection operation in accordance with mounting or dismounting of the sensor detected by the sensor detector.

8. The measuring device according to any one of claims 1-7, further comprising a forbiddance-cancelling condition determiner for determining whether or not a predetermined forbiddance-cancelling condition is satisfied,
wherein the transmission forbidder forbids transmission of the data to the communication device or to the monitoring device when the forbidding condition determiner determines that the predetermined forbidding condition is satisfied and the forbiddance-cancelling condition determiner determines that the predetermined forbiddance-cancelling condition is not satisfied.

9. A communication device for performing communication with a monitoring device for monitoring data received via a network in a monitoring system, the communication device comprising:
a data obtainer for obtaining measurement results measured by a measuring device as the data;
a transferer for sending the data to the monitoring device when the data obtainer obtains the data;
a forbidding condition determiner for determining whether or not a predetermined forbidding condition is satisfied; and
a transmission forbidder for forbidding the transferer from sending the data to the monitoring device when the forbidding condition determiner determines that the predetermined forbidding condition is satisfied.

10. The communication device according to claim 9, further comprising a service state checker for checking a communication service state,
wherein the forbidding condition determiner determines that the predetermined forbidding condition is satisfied when the service state checker finds that a predetermined service is to be performed.

11. The communication device according to claim 10, wherein the predetermined service is international roaming.

12. The communication device according to any one of claims 9-11, further comprising a forbiddance-cancelling condition determiner for determining whether or not a predetermined forbiddance-cancelling condition is satisfied,
wherein the transmission forbidder forbids the transferer from sending the data to the monitoring device when the forbidding condition determiner determines that the predetermined forbidding condition is satisfied and the forbiddance-cancelling condition determiner determines that the predetermined forbiddance-cancelling condition is not satisfied.

13. The communication device according to any one of claims 9-12, wherein: the monitoring device is provided for each of a plurality of kinds of data,
the communication device further comprises a data discriminator for discriminating among the plurality of kinds of data, and
the transferer sends the data to the monitoring device corresponding to the kind of the data discriminated by the data discriminator.

14. A monitoring system comprising:
the monitoring device; and
the measuring device as set forth in any one of claims 1-8 or the communication device as set forth in any one of claims 9-13.

15. A program for controlling a computer of a measuring device for measuring data, in a monitoring system including a monitoring device for monitoring the data received via a network, the program being designed to cause the computer to function as:
a data transmitter for sending the data to a communication device that performs communication with the monitoring device;
a forbidding condition determiner for determining whether or not a predetermined forbidding condition is satisfied; and
a transmission forbidder for forbidding transmission of the data to the communication device or to the monitoring device when the forbidding condition determiner determines that the predetermined forbidding condition is satisfied.

16. A program for controlling a computer of a communication device for performing communication with a monitoring device for monitoring data receivedvia a network in a monitoring system, the program being designed to cause the computer to function as:
a transferer for sending the data to the monitoring device upon receiving the data;
a forbidding condition determiner for determining whether or not a predetermined forbidding condition is satisfied; and
a transmission forbidder for forbidding the transferer from sending the data to the monitoring device when the forbidding condition determiner determines that the predetermined forbidding condition is satisfied.
